## Europäisches Patentamt

## European Patent Office

## Office européen des brevets

(11) Veröffentlichungsnummer : **0 079 441**
**B1**

(12) **EUROPÄISCHE PATENTSCHRIFT**

(45) Veröffentlichungstag der Patentschrift :
04.05.88

(51) Int. Cl.⁴ : **A 61 F   2/30**

(21) Anmeldenummer : **82108411.8**

(22) Anmeldetag : **11.09.82**

(54) **Endoprothese zum Ersatz stabförmiger Knochen.**

(30) Priorität : **30.09.81 DE 3138848**

(43) Veröffentlichungstag der Anmeldung :
**25.05.83 Patentblatt 83/21**

(45) Bekanntmachung des Hinweises auf die Patenterteilung : **04.05.88 Patentblatt 88/18**

(84) Benannte Vertragsstaaten :
**CH FR GB LI NL SE**

(56) Entgegenhaltungen :
**DE-A- 2 305 442**
**DE-B- 2 247 560**
**GB-A- 1 534 071**
**US-A- 4 016 874**
**US-A- 4 190 044**
**US-A- 4 237 875**
**US-A- 4 262 665**
**US-A- 4 293 962**

(73) Patentinhaber : **GMT Gesellschaft für medizinische Technik mbH**
**Holstenstrasse 2**
**D-2000 Hamburg 50 (DE)**

**Waldemar Link GmbH & Co**
**Barkhausenweg 10**
**D-2000 Hamburg 63 (DE)**

(72) Erfinder : **Keller, Arnold**
**An der Naherfurth 5**
**D-2061 Kaihude (DE)**
Erfinder : **Engelbrecht, Eckart**
**Andreasstrasse 33**
**D-2000 Hamburg 60 (DE)**
Erfinder : **Nieder, Elmar**
**Hinterdeich 117**
**D-2155 York (DE)**

(74) Vertreter : **Heldt, Gert, Dr. Dipl.-Ing.**
**Neuer Wall 59 III**
**D-2000 Hamburg 36 (DE)**

**Beschreibung**

Die Erfindung betrifft eine Endoprothese zum zumindest teilweisen Ersatz eines stabförmigen Knochens, die aus einem Stabteil und je einem mit dem Stabteil verbundenen Gelenkteil an den beiden Enden des Stabteils besteht.

Endoprothesen haben sich in der Praxis einen festen Platz für die Versorgung von Kranken gesichert, bei denen Knochendefekte im Regelfall nur an einem Ende eines stabförmigen Knochens auftreten. Der Einbau derartiger Endoprothesen mit Rücksicht auf die individuellen Erfordernisse eines bestimmten Patienten ist dadurch möglich, daß zum Ausgleich von Längenunterschieden die entsprechende Endoprothese mehr oder minder in den zu versorgenden Knochen eingesetzt wird.

Darüber hinaus werden aber auch Endoprothesen in den Fällen verwendet, in denen ein ganzer stabförmiger Knochen ersetzt werden muß. So ist ein Femurtotalersatz bekannt, bei dem ein totaler Femur durch eine Endoprothese ersetzt wird. In diesen Fällen besitzt der Stabteil an seinen beiden Enden je ein fest angesetztes Gelenkteil. Eine Möglichkeit zur Längenfeinanpassung oder zur nachträglichen Korrektur der Winkelstellung beider Gelenkteile zueinander ist nicht gegeben. Es muß daher jede Endoprothese für den jeweiligen Einzelfall individuell angefertigt werden. Die Länge der Endoprothese muß so bestimmt werden, daß die beiden Gelenkteile den individuellen Bedürfnissen eines Patienten entsprechend eingebaut werden können, ohne daß Längenverschiebungen ungewollt auftreten.

Derartige Längenverschiebungen führen im Regelfall dazu, daß der mit einer solchen Endoprothese versorgte Patient körperliche Schwierigkeiten in Kauf nehmen muß. Beispielsweise kann ein nicht richtig bemessener Ersatz für einen Femur dazu führen, daß das auf diese Weise versorgte Bein eines Patienten länger ist als das andere. Ferner bereitet die präoperative Vermessung der Längen, die herzustellen sind, mitunter Schwierigkeiten, so daß Fehler nicht immer ausgeschlossen werden können. Dies kann im Extremfall zur Unmöglichkeit des Einbaus in der operativen Situation führen.

Aufgabe der vorliegenden Erfindung ist es daher, die Endoprothese der einleitend genannten Art so zu verbessern, daß Schwierigkeiten beim Einbau der Endoprothese verhindert werden, die durch eine nicht ausreichende Bemessung der Prothese entstehen können. Darüber hinaus kann sich intraoperativ die Situation ergeben, daß das vorgefetigte, geplante Gelenkteil nicht implantierbar ist und ein anderes gewünscht wird. Diese Schwierigkeiten sollen ebenfalls verhindert werden.

Diese Aufgabe wird erfindungsgemäß dadurch gelöst, daß der Stabteil zur Anpassung an individuelle Verhältnisse aus mehreren, miteinander zu verbindenden oder miteinander verbundenen stabförmigen Teilen konfigurierbarer Länge besteht, die an ihren jeweils einander zugewandt angeordneten Enden Verbindungselemente aufweisen.

Weitere Ausgestaltungen gehen aus den abhängigen Ansprüchen hervor.

Durch diese Anpaßbarkeit mindestens eines der Teile ist es möglich, noch während der Operation die gesamte Endoprothese so zu wählen, daß sie den individuellen Bedürfnissen ihres Trägers exakt Rechnung tragen kann. Eine derartige Anpassung kann vor Beginn der operation durch eine entsprechende Operationsplanung außerordentlich schwierig sein, da die für die Bemessung der Endoprothese notwendigen röntgenologischen Aufnahmen die genauen anatomischen Abmessungen eines Knochens relativ ungenau wiedergeben. Die erfindungsgemäße Endoprothese kann noch während der Operation den jeweiligen Verhältnissen genau angepaßt werden, ohne daß die Operationszeit dadurch wesentlich verlängert wird. Durch eine entsprechende Bemessung und Auswahl der Form sowohl der Gelenke als auch des Stabteils ist eine Feinanpassung der benötigten Längen und der Lage möglich, in der die Endoprothese nach ihrem Einbau verlaufen soll. Ferner können die notwendigen Gelenkteile variiert werden.

Schwierig ist insbesondere die Versorgung solcher Knochen, die sowohl proximal als auch distal Defekte aufweisen. Das zwischen den Defekten liegende Knochenstück ist häufig so kurz, daß Endoprothesen an ihm nicht mehr befestigt werden können. Bisher war es notwendig, den Restknochen durch eine vollständige Endoprothese zu ersetzen.

Nach einer weiteren Ausbildung der Erfindung kann der Stabteil aus einer Mehrzahl von stabförmigen Teilen bestehen, die jeweils an ihren beiden Enden mit jeweils einem Verbindungselement versehen sind.

Diese stabförmigen Teile können vom Chirurgen so ausgewählt werden, daß die Verbindungsstellen an einer Stelle liegen, die für die Durchführung der Operation besonders günstig ist. Beispielsweise ist es denkbar, bei einem großen Defekt eines Röhrenknochens die Endoprothese mit ihrem Stabteil durch diesen hindurchzustecken und anschließend die beiden Gelenkteile mit dem Stabteil zu verbinden. In diesem Falle wird der Stabteil zweckmäßigerweise so ausgeführt, daß er innerhalb des Röhrenknochens einteilig verläuft, so daß Verbindungselemente zum Verbinden mit den anderen Teilen des Stabteils bzw. den Gelenkteilen lediglich außerhalb des Röhrenknochens vorhanden sind.

Auf diese Weise wird der Röhrenknochen einerseits mit einer inneren Schienung versehen und andererseits können die von dem Röhrenknochen zu übertragenden Kräfte nunmehr problemlos durch die Endoprothese übertragen werden. Diese operationstechnik hat insbesondere dann Vorteile, wenn der Rest des Röhrenknochens aus biomechanischen Gründen nicht mehr ausreicht, um in ihm beidseitig Teilendoprothesen zu befestigen, die den Anschluß zu dem benachbarten Knochen herstellen können. Beispielsweise ist es denkbar, durch die gesunden Teile eines Femurs eine totale Femur-Endoprothese hindurchzuführen, die einerseits mit einem Hüftgelenk und andererseits mit einem Kniegelenk verbunden ist.

Durch diese Ausbildung einer Durchsteckprothese wird soviel wie möglich erhaltenswerter Knochen belassen, sofern dieser nicht durch Infektionen, Trauma, Tumor usw. zerstört ist.

Auf diese Weise wird das Implantat so weit wie möglich knöchern gedeckt. Die erhaltenswerten Knochen bieten Ansatz für Sehnen und Muskeln. Soweit die Knochenteile erhaltenswerte Gelenkteile aufweisen, können diese weiterhin zur Ausführung ihrer Funktionen herangezogen werden. Insbesondere erweist es sich als zweckmäßig, das patellare Gleitlager zu erhalten, wenn beispielsweise das Femur durch eine Durchsteckprothese ersetzt wird.

Diese Durchsteckprothese kann den individuellen Bedürfnissen genau angepaßt werden, da die für den Anschluß des Femurs notwendigen Gelenkteile sowohl proximal als auch distal bezüglich des Mittelteils in bestimmten Winkelstellungen zueinander eingerichtet und fixiert werden können. Diese Anpaßbarkeit ist im Hinblick auf individuelle Unterschiede, die von Fall zu Fall auftreten können, von erheblicher Wichtigkeit. Ferner können individuell notwendige Gelenkteile aufgesetzt werden, im Falle eines Femurs proxImal ein Kopfabschluß oder eine Satteiprothese, distal ein Scharnierknie oder ein Rotationsknie.

Weitere Elnzelheiten der Erfindung ergeben sich aus der nachfolgenden ausführlichen Beschreibung und den beigefügten Zeichnungen, in denen eine bevorzugte Ausführungsform der Erfindung beispielsweise veranschaulicht ist.

In den Zeichnungen zeigen :

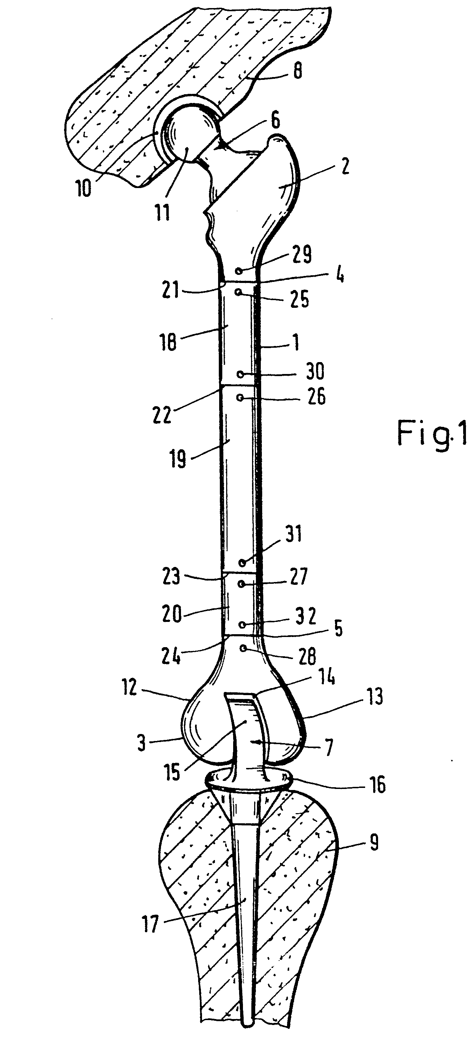

Fig. 1 : Eine Ansicht eines zusammengesetzten Bausatzes einer Endoprothese,

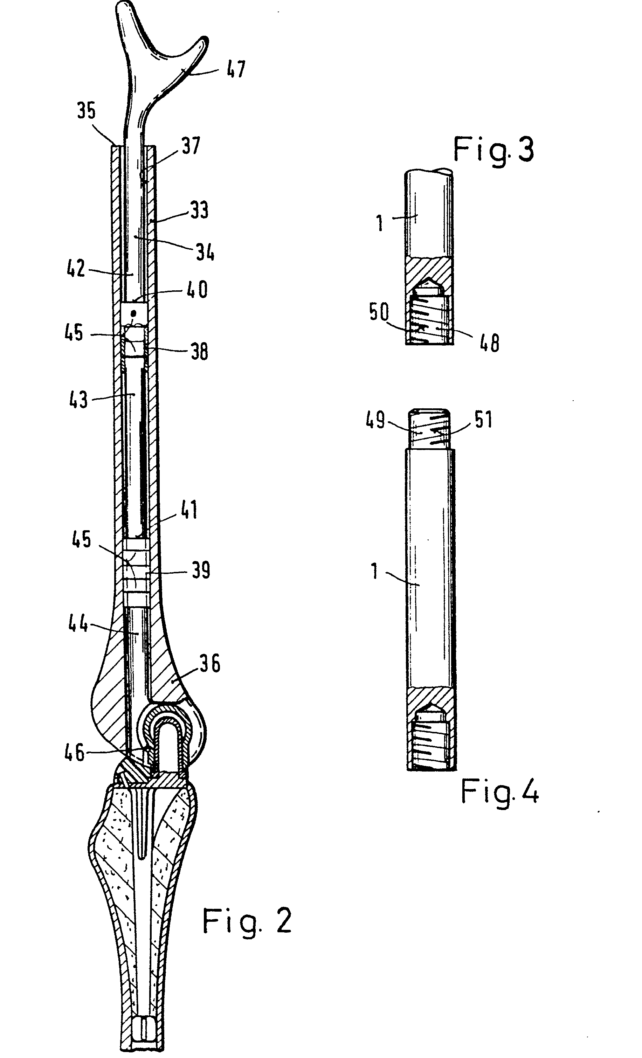

Fig. 2 : eine Ansicht einer in einen Knochen eingesetzten Endoprothese aus mehreren Bauteilen,

Fig. 3 : einen Schnitt durch einen Stabteil einer Endoprothese mit Gewindeanschluß,

Fig. 4 : einen Längsschnitt durch einen Zwischenteil einer Endoprothese mit Gewindeanschlüssen,

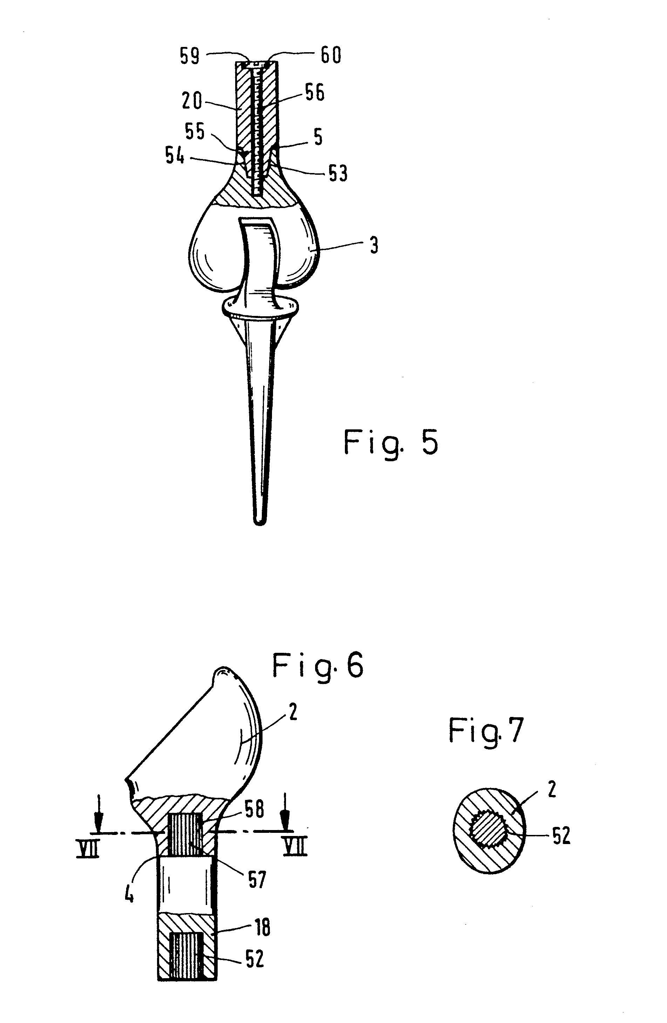

Fig. 5 : eine teilweise geschnittene Längsdarstellung einer konischen Verbindungsstelle,

Fig. 6 : eine teilweise geschnittene Längsdarstellung einer mit einer Zähnelung versehenen Verbindung und

Fig. 7 : einen Querschnitt entlang der Schnittlinie VII-VII in Figur 6.

Eine Endoprothese besteht im wesentlichen aus einem Stabteil 1 und zwei als Anschlußteil ausgebildeten Gelenkteilen 2, 3. Die Gelenkteile 2, 3 sind an zwei einander sich gegenüberliegenden Enden 4, 5 des Stabteils 1 befestigt. Die Gelenkteile 2, 3 sind als Lager 6, 7 ausgebildet, über die der Stabteil 1 mit benachbarten Knochen 8, 9 schwenkbar in Verbindung steht. Das am oberen Ende 4 ausgebildete Lager 6 ist als ein im Beckenknochen 8 ausgebildetes Hüftgelenk ausgebildet, dessen Pfanne 10 im Knochen 8 befestigt ist. In diese Pfanne 10 faßt ein Kopf 11 des Hüftgelenkes formschlüssig hinein, der mit dem Gelenkteil 2 fest verbunden ist.

Im Bereich des unteren Endes 5 ist das Lager 7 als ein Scharnier ausgebildet, dessen beide Seitenteile 12, 13 im Gelenkteil 3 ausgebildet sind. Zwischen diesen beiden Seitenteilen 12, 13 ist ein Schlitz 14 ausgebildet, in dem ein Mittelteil 15 schwenkbar um eine nicht dargestellte Achse gelagert ist, die in den Seitenteilen 12, 13 befestigt ist. An das Mittelteil 15 schließt sich in Richtung auf den Unterschenkelknochen 9 über einen Teller 16 ein Schaft 17 an, der im Knochen 9 befestigt ist.

Der Stabteil 1 ersetzt einen Oberschenkelknochen. Er ist aus mehreren stabförmigen Teilen 18, 19, 20 zusammengesetzt, die von verschiedener Länge sind. Diese stabförmigen Teile 18, 19, 20 sind untereinander und mit den Gelenkteilen 2, 3 über Steckverbindungen 21, 22, 23, 24 verbunden. Im Bereich dieser Steckverbindungen 21, 22, 23, 24 ist eines der beiden zu verbindenden Teile mit einem hülsenartigen Loch versehen, in das der andere Teil mit einem an seinem Ende vorgesehenen Zapfen hineinragt. Um ein Verdrehen der einzelnen Teile 18, 19 20 gegeneinander und gegenüber den Gelenkteilen 2, 3 zu verhindern, werden die Steckverbindungen jeweils mit Sicherungsstiften 25, 26, 27, 28 gesichert. Diese ragen durch entsprechende Sicherungslöcher hindurch, die im Bereich der Steckverbindungen 21, 22, 23, 24 durch die einzelnen Teile 18, 19, 20 hindurchgebohrt sind. Bei der Bemessung der Sicherungsstifte 25, 26, 27, 28 ist darauf zu achten, daß diese in ihrer Länge dem Durchmesser des Stabteiles 1 angepaßt sind, um zu verhindern, daß sie im eingebauten Zustand aus den sie aufnehmenden Löchern einseitig oder beidseitig herausragen.

Es ist auch möglich, die Steckverbindungen 21, 22, 23, 24 in der Weise herzustellen, daß sämtliche Teile 18, 19, 20 und die Gelenkteile 2, 3 an den einander zugewandten Enden mit hülsenartigen Bohrungen versehen werden. In diesem Falle werden in die entsprechenden Bohrungen lose Stifte hineingesteckt, die allerdings in den beiden sich gegenüber liegenden Enden der Teile 18, 19, 20 bzw. der Gelenkteile 2, 3 durch entsprechende Sicherungsstifte 29, 30, 31, 32 zusätzlich gesichert werden müssen. Diese Ausführungsform hat den Vorteil, daß die Anzahl der Kombinationsmöglichkeiten der Teile 18, 19, 20 vergrößert wird.

Es ist auch denkbar, aufgrund einer Operationsplanung mindestens einige der miteinander zu verbindenden Teile 18, 19, 20 so vorzufertigen, daß sie während der Operation nur noch zusammengesteckt zu werden brauchen. In manchen Fällen wird aber eine derartige Operationsplanung nicht möglich sein, so daß mit Hilfe von kurzen Teilen 20 noch während der Operation ein Längenausgleich vorgenommen werden muß, falls sich dieserals notwendig erweisen sollte.

Es ist auch möglich, eine in dieser Weise konzipierte Prothese als Durchsteckprothese 34 zu konzipieren. Diese dient dazu, durch einen defekten Knochen 33 hindurchgesteckt zu werden. Der Defekt an diesem Knochen 33 ist in solchen Fällen derartig umfangreich, daß eine Befestigung von Endoprothesen an seinen beiden Enden 35, 36 nicht möglich ist. Der defekte Knochen 33 ist nicht in der Lage, die von den Prothesen in ihn eingeleiteten Kräfte zu übertragen, ohne dabei weiter zerstört zu werden.

Zum Zwecke der Befestigung einer Durchsteckprothese 34 in einem Knochen 33 wird in diesen ein sich in Längsrichtung durch ihn erstreckendes Loch 37 gebohrt. Die Weite dieses Loches 37 ist so zu bestimmen, daß die Durchsteckprothese 34 mit ihren Steckverbindungen 38, 39 in das Loch 37 eingeführt werden kann. Dabei ist es denkbar, die Steckverbindungen 38, 39 mit Hülsen 40, 41 vorzunehmen, die über die jeweiligen Enden der einander benachbarten Teile 42, 43, 44 passend geschoben werden. Die Hülsen werden mit Sicherungsstiften 45 mit den jeweils in sie hineinragenden Teilen 42, 43, 44 verbunden.

Falls es sich bei dem defekten Knochen 33 um einen Oberschenkelknochen handelt, kann dieser an seinem unteren Ende 36 mit einem Rotationskniegelenk 46 versehen sein, während an seinem oberen Ende 35 eine Sattelprothese 47 vorgesehen ist, die eine unmittelbare Verbindung mit dem dem oberen Ende 35 benachbarten Beckenknochen ermöglicht.

Auch bei dieser Durchsteckprothese 34 können wesentliche Teile bereits vor Beginn der Operation vorgefertigt und miteinander verbunden werden.

Falls sich beim Einbau der Durchsteckprothese 34 in den Knochen 33 herausstellen sollte, daß die Durchsteckprothese 34 den individuellen Verhältnissen des Patienten besser angepaßt werden muß, ist es denkbar, auch im Rahmen der Durchsteckprothese 34 zusätzlich zu den längenkonfigurierbaren stabförmigen Teilen 42, 43, 44 Zwischenstücke zu verwenden, die einem Ausgleich von Längendifferenzen dienen.

Eine weitere Anpassung der Durchsteckprothese 34 an die individuellen Verhältnisse des Patienten ist dadurch möglich, daß die Gelenkteile 2, 3 proximal und distal an den stabförmigen Teilen in Bestimmten Winkelstellungen zueinander eingerichtet werden und in diesen Winkelstellungen fixierbar sind. Zu diesem Zwecke kann eine Zähnelung 52 vorgesehen sein, die einen möglichst kleinen Modul aufweisen sollte. Während die Zähnelung eines der beiden aneinander anzuschließenden Teile 18 im Bereich seines Einem Gelenkteil 2 zugewandten Endes 4 auf einem sich im Zentrum dieses Endes 4 erhebenden zylinderartigen Vorsprung 57 vorgesehen ist, ist im benachbarten Gelenkteil 2 eine an entsprechender Stelle vorgesehene Mittelbohrung 58 vorgesehen, in die der Vorsprung 57 hineinragt. Der Vorsprung 57 ist an seiner zylindrischen Außenfläche mit einer Zähnelung 52 versehen, der eine Zähnelung entspricht, die sich auf den zylindrischen Innenflächen der Mittelbohrung 58 erhebt.

Die Module der Zähnelung 52 sind so gewählt, daß die beiden Zähnelungen ineinander geführt werden können, wenn das Gelenkteil 2 auf das Teil 18 aufgesteckt wird. Je nach den beim Patienten vorgefundenen anatomischen Verhältnissen kann eine Winkelstellung des Gelenkteil 2 gegenüber dem Teil 18 gewählt werden. In dieser Winkelstellung rasten die Zähnelungen 52 ineinander ein, so daß das Gelenkteil 2 gegenüber dem Teil 18 nicht verdrehbar ist.

Eine ähnliche Justiermöglichkeit ist dadurch möglich, daß die miteinander zu verbindenden Teile 2, 3 ; 18, 19, 20 ; 42, 43,44 über konische Verbindungsstellen 53 miteinander verbunden werden. Dabei kann sich beispielsweise auf dem Ende 5 eines Teiles 20 ein Außenkonus 54 erheben, der hinsichtlich der Winkelstellung seiner Konusflächen mit einem Innenkonus 55 übereinstimmt, der in das benachbarte Ende eines Gelenkteils 3 eingebracht ist. Die Konusflächen des Außenkonus 54 werden auf die des Innenkonus 55 mit Hilfe einer Spannschraube 56 gedrückt, die sich in Längsrichtung durch das Teil 20 erstreckt und mit einem im Gelenkteil 3 vorgesehenen Innengewinde zusammenarbeitet. Mit Hilfe dieser Spannschraube 56, die im Teil 20 frei drehbar ist, werden die Konusflächen so fest aufeinander gepreßt, daß eine Verdrehung der beiden Teile 20, 3 gegeneinander nicht möglich ist.

Das mit weiteren Teilen 18, 19 und einem weiteren Gelenkteil 2 verbundene Teil 20 kann seinerseits mit dem benachbarten Teil 19 über Konusverbindung verbunden sein. In diesem Falle stützt sich die Spannschraube 56 mit ihrem Kopf 59 auf dem Boden 60 eines nicht dargestellten Innenkonus ab, mit dem ein entsprechender Außenkonus des benachbarten Teiles 19 zusammenarbeitet. Darüber hinaus ist es aber auch denkbar, die Teile 19, 20 auf andere Weise miteinander zu verbinden, beispielsweise durch Sicherungsstifte 29, 30, 31, 32. Mit Hilfe der Konen 54, 55 ist eine stufenlose Anpaßbarkeit der Winkelstellung an die jeweils aufgefundenen anatomischen Verhältnisse eines Patienten möglich. Knochenteile müssen nicht deswegen entfernt werden, damit die Durchsteckprothese 34 in den jeweiligen Knochenbau des Patienten eingefügt werden kann. Auf diese Weise wird ein Maximum erhaltenswerter Knochen belassen, sofern dieser nicht durch Infektion, Trauma, Tumor usw. zerstört ist. Die Erhaltung des Knochens ist wichtig, um

1. das Implanat knöchern zu decken,

2. einen Ansatz für Sehnen und Muskeln zu bieten und

3. falls der erhaltene Knochen erhaltenswerte Gelenkteile beinhaltet, diese weiterhin so weit wie möglich zu nutzen.

Insbesondere im Bereich des patellaren Gleitlagers ergeben sich erhebliche Vorteile durch die Erhaltung natürlicher Knochenteile.

Schließlich ist es denkbar, die einzelnen Teile 18, 19, 20 ; 42, 43, 44 über Schraubverbindungen 48, 49 miteinander zu verbinden. In diesem Falle werden zwei Stabteile 1 zusammengeschraubt ; während der eine Stabteil an seinem einen Ende ein Innengewinde 50 aufweist, ist der ihm benachbarte Stabteil mit seinem ihm zugewandten Ende mit einem Außengewinde 51 versehen. Die beiden Gewinde 50, 51 sind hinsichtlich ihrer Länge so eingerichtet, daß das Außengewinde 51 vollkommen im Innengewinde verschwindet, wenn die beiden Teile des Stabteils 1 fest miteinander verschraubt sind. Gleichzeitig können die beiden Gewinde 50, 51 als Feingewinde ausgeführt werden, das eine selbsthemmende Wirkung zeigt, wenn die beiden Teile fest miteinander verschraubt werden. Darüber hinaus kann aber auch noch eine Sicherung gegen Verdrehen beispielsweise mit zusätzlichen Sicherungsstiften vorgenommen werden.

Um einen gleichmäßigen Längenausgleich herbeiführen zu können, ist es darüber hinaus denkbar, die einzelnene Teile 18, 19, 20 ; 42, 43, 44 einzeln oder in ihrer Gesamtheit als Teleskopteile auszubilden. Diese können je nach Bedarf weiter oder weniger weit auseinander gezogen und anschließend durch geeignete Maßnahmen, beispielsweise Sicherungsstifte gegen Verschieben gesichert werden.

Zweckmäßigerweise wird die Endoprothese in Form von Bausätzen hergestellt und geliefert. Diese Bausätze enthalten sämtliche vom Anwender gewünschten Einzelteile. Darüber hinaus sind in dem Bausatz Ausgleichsstücke vorgesehen, deren Länge auf übliche Maßtoleranzen abgestellt sind. Diese Ausgleichsstücke können vom Chirurgen je nach den individuellen Verhältnissen, die er beim Patienten vorfindet, angewendet werden.

Die Endoprothese ist in allen Fällen einsetzbar, in denen stabförmige Knochen Defekte aufweisen. In ihrer Version als Durchsteckprothese 34 ist sie insbesondere für Röhrenknochen anwendbar. Sie kann daher auch im Bereich der Armknochen Verwendung finden ; als Durchsteckprothese für einen Oberarmknochen ist sie einerseits mit einem Schultergelenk und andererseits mit einem Ellenbogengelenk versehen.

Die Gelenkteile 2,3 können im Rahmen des als Durchsteckprothese 34 zu verwendenden Bausatzes bereits vorgefertigt sein. Dabei ist es möglich, daß das distale Gelenkteil 3 vorgefertigt als gleichzeitiger Ersatz der Condylen montierbar ist. Auf diese Weise kann wahlweise mit den Teilen 18, 19, 20 ; 42, 43, 44 eine Kniegelenk-Endoprothese, welcher Art auch immer, oder aber eine Kniegelenk-Endoprothese mit Condylenersatz, welcher Art auch immer (Rotations- oder Scharnier-Endoprothese) montiert werden. Es ist jedoch auch möglich, daß das distale Gelenkteil 3, nämlich die Scharnier- oder Rotations-Endoprothese wahlweise mit einem möglicherweise aufschiebbaren Condylenersatz montiert wird, daß ferner im Bedarfsfalle ein patellares Gleitlager eingesetzt werden kann, und daß schließlich sowohl die Condylen als auch das patellare Gleitlager ersetzt werden können. Auf diese Weise erhält der Chirurg ein optimales Bausatzsystem, das erhebliche Kosteneinsparungen sowohl bei der Montage als auch bei der Herstellung mit sich bringt. Beispielsweise ist es denkbar, eine Durchsteckprothese 34 zu fertigen, die distal das Gelenk 3 aufweist. Im Bedarfsfalle können dazu entweder die Condylen oder das patellare Gleitlager oder beide gemeinsam zusätzlich montiert werden.

Als Material für die Herstellung der Prothese kommt im wesentlichen Metall in Betracht. Zu denken ist insbesondere an eine Kobalt-Chrom Molybdän-Legierung, die im Bereich der medizinischen Technik als Vitallium (eingetragenes Warenzeichen) bekannt ist. Darüber hinaus können die Teile aber auch aus einer Titanlegierung hergestellt werden. In diesem Falle wird der Kopf 11 eines Hüftgelenkes aus Keramik gefertigt werden, während die Pfanne 10 bzw. andere Gleitlager aus Polyäthylen bestehen.

**Patentansprüche**

1. Endoprothese zum zumindest teilweisen Ersatz eines stabförmigen Knochens, die aus einem Stabteil (1) und je einem mit dem Stabteil verbundenen Gelenkteil (2, 3) an den beiden Enden (4, 5) des Stabteils besteht, dadurch gekennzeichnet, daß der Stabteil (1) zur Anpassung an individuelle Verhältnisse aus mehreren, miteinander zu verbindenden oder miteinander verbundenen stabförmigen Teilen (18, 19, 20 ; 42, 43, 44) konfigurierbarer Länge besteht, die an ihren jeweils einander zugewandt angeordneten Enden Verbindungselemente (22, 23, 26, 27, 30, 31 ; 40, 41, 45) aufweisen.

2. Endoprothese nach Anspruch 1, dadurch gekennzeichet, daß die Verbindungselemente (22, 26, 30, 23, 27, 31 ; 40, 41, 45) als Steckverbindungen (22, 23, 38, 39) ausgestaltet sind, wobei ein Ende jedes stabförmigen Teils (18, 19, 20) als Hülse und das diesem Ende zugewandte Ende des benachbarten stabförmigen Teils als in die Hülse hineinragender Zapfen ausgebildet ist oder wobei die Steckverbindungen (38, 39) mit Hülsen (40, 41) vorgenommen werden, die über die jeweiligen Enden der einander benachbarten stabförmigen Teile (42, 43, 44) passend geschoben werden.

3. Endoprothese nach Anspruch 2, dadurch gekennzeichnet, daß der Zapfen in der Hülse gesichert ist oder die in die Hülse (40, 41) geschoberen Enden der einander benachbarten stabförmigen Teile (42, 43, 44) in der Hülse (40, 41) gesichert sind.

4. Endoprothese nach Anspruch 3, dadurch gekennzeichnet, daß zur Sicherung Sicherungsstifte (26, 27, 45) vorgesehen sind, die jeweils durch den Zapfen und die Hülse oder durch die Hülse und die Enden der benachbarten Teile hindurchgreift.

5. Endoprothese nach einem der Ansprüche 2 bis 4, dadurch gekennzeichnet, daß der Zapfen mit der Hülse verschraubt (48, 49, 50, 51) ist.

6. Endoprothese nach einem der Ansprüche 1 bis 5, dadurch gekennzeichnet, daß einer der beiden Gelenkteile (2, 3) als ein Hüftgelenk ausgebildet ist.

7. Endoprothese nach einem der Ansprüche 1 bis 5, dadurch gekennzeichnet, daß einer der beiden Gelenkteile (2, 3) als eine sich in einem Beckenknochen unmittelbar abstützende Sattelprothese (47) ausgebildet ist.

8. Endoprothese nach einem der Ansprüche 1 bis 5, dadurch gekennzeichnet, daß einer der beiden Gelenkteile (2, 3) als eine Kniegelenkendoprothese ausgebildet ist.

9. Endoprothese nach einem der Ansprüche 1 bis 5, dadurch gekennzeichnet, daß einer der beiden Gelenkteile (2, 3) als eine Scharnierendoprothese ausgebildet ist.

10. Endoprothese nach einem der Ansprüche 1 bis 5, dadurch gekennzeichnet, daß einer der beiden Gelenkteile (2, 3) als eine Rotationsendoprothese ausgebildet ist.

11. Endoprothese nach einem der Ansprüche 1 bis 5, dadurch gekennzeichnet, daß einer der beiden Gelenkteile (2, 3) als eine Schultergelenkendoprothese ausgebildet ist.

12. Endoprothese nach einem der Ansprüche 1 bis 5, dadurch gekennzeichnet, daß einer der beiden Gelenkteile (2, 3) als eine Ellenbogengelenkendoprothese ausgebildet ist.

13. Endoprothese nach einem der Ansprüche 1 bis 12, dadurch gekennzeichnet, daß mindestens zwei der miteinander zu verbindenden Teile (18, 19, 20 ; 42, 43, 44) eine vorgefertigte Einheit bilden.

14. Endoprothese nach einem der Ansprüche 1 bis 13, dadurch gekennzeichnet, daß sämtliche stabförmigen Teile und Gelenkteile (2, 3 ; 18, 19, 20 ; 42, 43, 44) eine vorgefertigte Einheit bilden, deren Zerlegung in einzelne Teile (18, 19, 20 ; 42 43 44 ; 2, 3) zu Zwecken des vorteilhaften Einbaus vorgesehen ist.

15. Endoprothese nach einem der Ansprüche 1 bis 14, dadurch gekennzeichnet, daß die stabförmigen Teile und Gelenkteile (2, 3 ; 18, 19, 20 ; 42, 43, 44) aus Metall sind.

16. Endoprothese nach Anspruch 15, dadurch gekennzeichnet, daß die stabförmigen Teile und Gelenkteile (2, 3 ; 18, 19, 20 ; 42, 43, 44) aus einer Kobalt-Chrom-Molybdän-Legierung bestehen.

17. Endoprothese nach Anspruch 15, dadurch gekennzeichnet, daß die stabförmigen Teile und Gelenkteile (2, 3 ; 18, 19, 20 ; 42, 43, 44) aus Vitallium (eingetr. Warenzeichen) bestehen.

18. Endoprothese nach Anspruch 15, dadurch gekennzeichnet, daß die stabförmigen Teile und Gelenkteile (2, 3 ; 18, 19, 20 ; 42, 43, 44) aus einer Titanlegierung und das Lager (6) des Gelenkteils (2) aus einem Kopf (11) aus Keramik bestehen, der auf einer aus Polyäthylen bestehenden Pfanne (10) gleitet.

19. Endoprothese nach einem der Ansprüche 1 bis 18, dadurch gekennzeichnet, daß sie als eine Durchsteckprothese (34) ausgebildet ist, deren Stabteil (42, 43, 44) durch einen defekten Knochen (33) hindurchsteckbar, mit diesem verbindbar und anschließend mit den Gelenkteilen (2, 3) versehbar ist.

20. Endoprothese nacheinem der Ansprüche 1 bis 19, dadurch gekennzeichnet, daß die Gelenkteile (2, 3) mindestens einseitig bezüglich des Stabteils (1) individuell einrichtbar angeordnet sind.

21. Endoprothese nach einem der Ansprüche 1 bis 20, dadurch gekennzeichnet, daß der Stabteil (1) und die Gelenkteile (2, 3) untereinander formschlüssig geführt und um ihre gemeinsame Längsachse zueinander in bestimmten Winkelstellungen justierbar sind.

22. Endoprothese nach einem der Ansprüche 1 bis 21, dadurch gekennzeichnet, daß die Stab- und Gelenkteile (1, 2, 3) über entsprechend ausgewählte stabförmige Teile (18, 20) in ihrer Länge in kleinen Schritten einstellbar sind.

23. Endoprothese nach einem der Ansprüche 1 bis 22, dadurch gekennzeichnet, daß zwischen den Gelenkteilen (2, 3) und den Teilen (18, 19, 20 ; 42, 43, 44) des Stabteils (1) mit einem kleinen Modul versehene Zähnelungen (52) vorgesehen sind.

24. Endoprothese nach einem der Ansprüche 1 bis 23, dadurch gekennzeichnet, daß zwischen dem Stabteil (1) und den Gelenkteilen (2, 3) sowie zwischen den Teilen (18, 19, 20 ; 42, 43, 44) des Stabteils (1) konische Verbindungsstellen (53) vorgesehen sind.

25. Endoprothese nach Anspruch 24, dadurch gekennzeichnet, daß im Bereich der konischen Verbindungsstellen (53) ein Außenkonus (54) in einen benachbarten Innenkonus (55) hineinragt und die beiden Konen (54, 55) sich mit Hilfe einer sie in Längsrichtung durchdringenden Spannschraube (56) gegenseitig beaufschlagen.

26. Endoprothese nach einem der Ansprüche 1 bis 25, dadurch gekennzeichnet, daß einer der beiden Gelenkteile (2, 3) mit einem Condylenersatz versehen ist.

27. Endoprothese nach Anspruch 26, dadurch gekennzeichnet, daß der Condylenersatz auf den Gelenkteil (2, 3) aufgeschoben ist.

28. Endoprothese nach einem der Ansprüche 1 bis 27, dadurch gekennzeichnet, daß einer der beiden Gelenkteile (2, 3) mit einem patellaren Gleitlager versehen ist.

Claims

1. An endoprosthesis for at least partial replacement of a barshaped bone, the endoprosthesis comprising a bar portion (1) and a respective joint portion (2, 3), connected to the bar portion, at each of the two ends (4, 5) of the bar portion, characterised in that, for the purposes of adaptation to individual conditions, the bar portion (1) comprises a plurality of bar-shaped portions (18, 19, 20 ; 42, 43, 44) of configurable length, which bar-shaped portions are to be connected together or are connected together and have connecting elements (22, 23, 26, 27, 30, 31 ; 40, 41, 45) at their respective ends which are arranged towards each other.

2. An endoprosthesis according to claim 1 characterised in that the connecting elements (22, 26, 30, 23, 27, 31 ; 40, 41, 45) are in the form of push-in connections (22, 23, 38, 39), wherein one end of each bar-shaped portion (18, 19, 20) is in the form of a sleeve and the end, which is towards said end, of the adjacent bar-shaped poition is in the form of a pin portion which projects into the sleeve, or wherein the push-in connections (38, 39) are made with sleeves (40, 41) which are suitably pushed over the respective ends of the mutually adjacent bar-shaped portions (42, 43, 44).

3. An endoprosthesis according to claim 2 characterised in that the pin portion is secured in the sleeve or the ends, which are pushed into the sleeve (40, 41), of the mutually adjacent bar-shaped portions (42, 43, 44) are secured in the sleeve (40, 41).

4. An endoprosthesis according to claim 3 characterised in that securing pins (26, 27, 45) are provided for securing purposes, the securing pins respectively engaging through the pin portion and the sleeve or through the sleeve and the ends of the adjacent portions.

5. An endoprosthesis according to one of claims 2 to 4 characterised in that the pin portion is screwed (48, 49, 50, 51) to the sleeve.

6. An endoprosthesis according to one of claims 1 to 5 characterised in that one of the two joint portions (2, 3) is in the form of a hip joint.

7. An endoprosthesis according to one of claims 1 to 5 characterised in that one of the two joint portions (2, 3) is in the form of a saddle prosthesis (47) which is supported directly in a pelvic bone.

8. An endoprosthesis according to one of claims 1 to 5 characterised in that one of the two joint portions (2, 3) is in the form of a knee joint endoprosthesis.

9. An endoprosthesis according to one of claims 1 to characterised in that one of the two joint portions (2, 3) is in the form of a hinge endoprosthesis.

10. An endoprosthesis according to one of claims 1 to 5 characterised in that one of the two joint portions (2, 3) is in the form of a rotation endoprosthesis.

11. An endoprosthesis according to one of claims 1 to 5 characterised in that one of the two joint portions (2, 3) is in the form of a shoulder joint endoprosthesis.

12. An endoprosthesis according to one of claims 1 to 5 characterised in that one of the two joint portions (2, 3) is in the form of an elbow joint endoprosthesis.

13. An endoprosthesis according to one of claims 1 to 12 characterised in that at least two of the portions (18, 19, 20 ; 42, 43, 44) which are to be connected together form a prefabricated unit.

14. An endoprosthesis according to one of claims 1 to 13 characterised in that all bar-shaped portions and joint portions (2, 3 ; 18, 19, 20 ; 42, 43, 44) form a prefabricated unit which can be broken down into individual portions (18, 19, 20 ; 42, 43, 44 ; 2, 3) for the purposes of advantageous fitting.

15. An endoprosthesis according to one of claims 1 to 14 characterised in that the bar-shaped portions and joint portions (2, 3 ; 18, 19, 20 ; 42, 43, 44) are of metal.

16. An endoprosthesis according to claim 15 characterised in that the bar-shaped portions and joint portions (2, 3 ; 18, 19, 20 ; 42, 43, 44) comprise a cobalt-chromium-molybdenum alloy.

17. An endoprosthesis according to claim 15 characterised in that the bar-shaped portions and joint portions (2, 3 ; 18, 19, 20 ; 42, 43, 44) comprise Vitallium (registered trade mark).

18. An endoprosthesis according to claim 15 characterised in that the bar-shaped portions and joint portions (2, 3 ; 18, 19, 20 ; 42, 43, 44) comprise a titanium alloy and the bearing (6) of the joint portion (2) comprises a head (11) of ceramic, which slides on a socket (10) consisting of polyethylene.

19. An endoprosthesis according to one of claims 1 to 18 characterised in that it is in the form of a through-fitting prosthesis (34) whose bar portion (42, 43, 44) can be fitted through a defective bone (33), can be connected thereto, and can then be provided with the joint portions (2, 3).

20. An endoprosthesis according to one of claims 1 to 19 characterised in that the joint portions (2, 3) are arranged to be individually adjustable at least at one end relative to the bar portion (1).

21. An endoprosthesis according to one of claims 1 to 20 characterised in that the bar portion (1) and the joint portions (2, 3) are guided positively relative to each other and can be adjusted relative to each other about their common longitudinal axis in given angular positions.

22. An endoprosthesis according to one of claims 1 to 21 characterised in that the bar and joint portions (1, 2, 3) are adjustable in their length in small steps by way of suitably selected bar-shaped portions (18, 20).

23. An endoprosthesis according to one of claims 1 to 22 characterised in that serrations (52) which have a small modulus are provided between the joint portions (2, 3) and the parts (18, 19, 20 ; 42, 43, 44) of the bar portion (1).

24. An endoprosthesis according to one of claims 1 to 23 characterised in that conical connecting means (53) are provided between the bar portion (1) and the joint portions (2, 3) and between the parts (18, 19, 20 ; 42, 43, 44) of the bar portion (1).

25. An endoprosthesis according to claim 24 characterised in that in the region of the conical connecting means (53) a male cone (54) projects into an adjacent female cone (55) and the two cones (54, 55) bear against each other by means of a clamping screw (56) which passes through them in the longitudinal direction.

26. An endoprosthesis according to one of clains 1 to 25 characterised in that one of the two joint portions (2, 3) is provided with a replacement condyle.

27. An endoprosthesis according to claim 26 characterised in that the replacement condyle is pushed on to the joint portion (2, 3).

28. An endoprosthesis according to one of claims 1 to 27 characterised in that one of the two joint portions (2, 3) is provided with a patellar sliding bearing.

**Revendications**

1. Endoprothèse destinée à remplacer au moins partiellement un os long, qui est composée d'une partie tige (1) et d'une partie articulation (2, 3) assemblée à la partie tige à chacune des deux extrémités (4, 5) de la partie tige, caractérisée en ce que, pour permettre l'adaptation aux conditions individuelles, la partie tige (1) est composée de plusieurs parties (18, 19, 20 ; 42, 43, 44) en forme de tige de longueur réglable, qui doivent être assemblées les unes aux autres ou sont assemblées les unes aux autres et qui présentent des éléments d'assemblage (22, 23, 26, 27, 30, 31 ; 40, 41, 45) à leurs extrémités qui sont dirigées les unes vers les autres.

2. Endoprothèse selon la revendication 1, caractérisée en ce que les éléments d'assemblage (22, 26, 30, 23, 27, 31 ; 40, 41, 45) sont constitués par des assemblages à emboîtement (22, 23, 38, 39), une extrémité de chaque partie en forme de tige (18, 19, 20) présentant la forme d'un manchon et l'extrémité de la partie en forme de tige adjacente qui est dirigée vers cette extrémité étant constituée par un tourillon qui s'engage dans le manchon, où les assemblages à emboîtement (38, 39) sont réalisés à l'aide de manchons (40, 41) qui sont emmanchés exactement sur les extrémités correspondantes des parties en forme de tiges (42, 43, 44) qui sont mutuellement adjacentes.

3. Endoprothèse selon la revendication 2, caractérisée en ce que le tourillon est bloqué dans le manchon où les extrémités des parties en forme de tiges (42, 43, 44) mutuellement adjacentes qui sont enfoncées dans le manchon (40, 41) sont bloquées dans le manchon (40, 41).

4. Endoprothèse selon la revendication 3, caractérisée en ce que, pour l'arrêt des pièces, il est prévu des goupilles de blocage (26, 27, 45) qui sont enfilées à travers le tourillon et le manchon ou à travers le manchon et les extrémités des éléments voisins.

5. Endoprothèse selon une des revendications 2 à 4, caractérisée en ce que le tourillon est vissé au manchon (48, 40, 50, 51).

6. Endoprothèse selon une des revendications 1 à 5 caractérisée en ce que l'une des deux parties articulations (2, 3) est constituée par une articulation de hanche.

7. Endoprothèse selon une des revendications 1 à 5, caractérisée en ce qu'une des deux parties articulations (2, 3) est constituée par une prothèse en selle (47) qui prend directement appui dans un os du bassin.

8. Endoprothèse selon une des revendications 1 à 5, caractérisée en ce qu'une des deux parties articulations (2, 3) est constituée par une endoprothèse d'articulation de genoux.

9. Endoprothèse selon une des revendications 1 à 5, caractérisée en ce qu'une des deux parties articulations (2, 3) est constituée par une endoprothèse d'articulation à charnière.

10. Endoprothèse selon une des revendications 1 à 5, caractérisée en ce qu'une des deux parties articulations (2, 3) est constituée par une endoprothèse d'articulation de rotation.

11. Endoprothèse selon une des revendications 1 à 5, caractérisée en ce qu'une des deux parties articulations (2, 3) est constituée par une endoprothèse d'articulation d'épaule.

12. Endoprothèse selon une des revendications 1 à 5, caractérisée en ce qu'elle est constituée par une endoprothèse d'articulation de coude.

13. Endoprothèse selon une des revendications 1 à 12 caractérisée en ce qu'au moins deux des parties (18, 19, 20 ; 42, 43, 44) qu'il s'agit de relier les unes aux autres, forment une unité préfabriquée.

14. Endoprothèse selon une des revendications 1 à 13, caractérisée en ce que toutes les parties en forme de tige et parties articulations (2, 3 ; 18, 19, 20 ; 42, 43, 44) forment une unité préfabriquée dont la décomposition en éléments individuels (18, 19, 20 ; 42, 43, 44 ; 2, 3) est prévue pour permettre un mode avantageux de montage.

15. Endoprothèse selon une des revendications 1 à 14, caractérisée en ce que les parties en forme de tige et les parties articulations (2, 3 ; 18, 19, 20 ; 42, 43, 44) sont métalliques.

16. Endoprothèse selon la revendication 15, caractérisée en ce que les parties en forme de tige et les parties articulations (2, 3 ; 18, 19, 20 ; 42, 43, 44) sont faites d'un alliage cobalt-chrome-molybdène.

17. Endoprothèse selon la revendication 15, caractérisée en ce que les parties en forme de tige et les parties articulations (2, 3 ; 18, 19, 20 ; 42, 43, 44) sont faites de vitallium (marque déposée).

18. Endoprothèse selon la revendication 15, caractérisée en ce que les parties en forme de tige et les parties articulations (2, 3 ; 18, 19, 20 ; 42, 43, 44) sont faites d'un alliage de titane et la portée (6) de la partie articulation (2) d'une tête (11) en céramique qui glisse sur une cuvette (10) en polyéthylène.

19. Endoprothèse selon l'une des revendications 1 à 18, caractérisée en ce qu'elle est constituée par une prothèse traversante (34) dont la partie tige (42, 43, 44) peut être enfilée à travers un os défectueux (33), assemblée à cet os et munie ensuite de parties articulations (2, 3).

20. Endoprothèse selon l'une des revendications 1 à 19, caractérisée en ce que les parties articulations (2, 3) sont agencées de façon à pouvoir être orientées individuellement par rapport à la partie tige (1), au moins d'un côté.

21. Endoprothèse selon une des revendications 1 à 20, caractérisée en ce que la partie tige (1) et les parties articulations (2, 3) sont guidées les unes par rapport aux autres par des liaisons par sûreté de forme et qu'elles peuvent être réglées les unes par rapport aux autres dans des positions angulaires déterminées par rotation autour de leur axe longitudinal commun.

22. Endoprothèse selon une des revendications 1 à 21, caractérisée en ce que les parties tiges et les parties articulations (1, 2, 3) sont réglables en longueur par petits pas, par l'intermédiaire de parties en forme de tige (18, 20) sélectionnées en conséquence.

23. Endoprothèse selon une des revendications 1 à 22, caractérisée en ce que des dentures (52) à petit module sont prévues entre les parties articulations (2, 3) et les éléments (18, 19, 20 ; 42, 43, 44) de la partie tige (1).

24. Endoprothèse selon une des revendications 1 à 23, caractérisée en ce que des zones d'assemblage à cône (53) sont prévues entre la partie tige (1) et les parties articulations (2, 3) ainsi qu'entre les éléments (18, 19, 20 ; 42, 43, 44) de la partie tige 1.

25. Endoprothèse selon la revendication 24, caractérisée en ce que, dans la région des zones d'assemblage à cône (53), un cône extérieur (54) pénètre dans un cône intérieur (55) adjacent et les deux cônes (54, 55) sont en appui l'un contre l'autre sous l'action d'une vis de serrage (56) qui les traverse dans la direction longitudinale.

26. Endoprothèse selon une des revendications 1 à 25, caractérisée en ce qu'une des deux parties articulations (2, 3) est munie d'une prothèse de condyle.

27. Endoprothèse selon la revendication 26, caractérisée en ce que la prothèse de condyle est emboîtée sur la partie articulation (2, 3).

28. Endoprothèse selon une des revendications 1 à 27, caractérisée en ce qu'une des deux parties articulations (2, 3) est munie d'une portée de glissement rotulaire.

0 079 441

Fig.1

0 079 441

Fig. 2

Fig. 3

Fig. 4

2

Fig. 5

Fig. 6

Fig. 7